# EUROPEAN PATENT APPLICATION

(11) **EP 4 610 716 A1**
(43) Date of publication of application: **03.09.2025**
(21) Application number: 23882768.7
(22) Date of filing: 27.10.2023
(51) Int. Cl.: G02C 7/02, G02C 11/00, A61N 5/06, A61F 9/007

(54) **METHOD FOR TREATING AND INHIBITING PROGRESS OF RETINAL DEGENERATION USING VIOLET LIGHT, AND DEVICE USED IN SAID METHOD**

(30) Priority: 28.10.2022 JP 2022172849
(71) Applicant: Tsubota Laboratory, Inc., Tokyo 160-8582 (JP)
(72) Inventor: TSUBOTA, Kazuo, Tokyo 160-8582 (JP); BAN, Norimitsu, Tokyo 160-8582 (JP); KURIHARA, Toshihide, Tokyo 160-8582 (JP)
(74) Representative: Plasseraud IP
(86) International application number: PCT/JP2023/038940
(87) International publication number: WO 2024/090566

(57) **Abstract**

The present invention provides a method for treating a retinal degeneration such as an age-related macular degeneration and a hereditary retinal degeneration by light stimulation in which light of a specific wavelength region such as violet light is pulsed at a specific frequency, and an apparatus used for the same. In the present invention, by irradiating a living body with light of a specific wavelength region such as violet light, at least one symptom selected from a group consisting of low vision, metamorphopsia, central scotoma, and complete loss of vision is improved or suppressed, and treatment and/or prophylaxis of the retinal degeneration such the age-related macular degeneration and the hereditary retinal degeneration is carried out.

## Description

### Technical Field

The present invention relates to a method for treating a disorder by light stimulation and an apparatus used for the same, and more particularly, relates to a method for treating a retinal degeneration such as an age-related macular degeneration and a hereditary retinal degeneration by light stimulation in which light of a specific wavelength region such as violet light is continuously irradiated or pulsed at a specific frequency, and an apparatus used for the same.

### Background Art

Causes of the retinal degeneration include causes such as the age-related macular degeneration and the hereditary retinal degeneration. The age-related macular degeneration is ranked high among causes of loss of vision in developed countries, and there is an exudative type which is characterized by choroidal neovascular of a macular region which is a central portion of retina and an atrophic type which is characterized by geographic atrophy of macular region and a loss of photoreceptor cells associated with geographic atrophy of macular region. Although intravitreal injection of anti-vascular endothelial growth factor (VEGF) antibody has been used as a standard therapy for the exudative age-related macular degeneration, the permanent cure is difficult, and it is necessary to administer it repeatedly over a long period of time. Regarding the exudative age-related macular degeneration, an invasive treatment (intravitreal injection), and complications (such as intraocular inflammation) and side effects (such as cerebral infarction) associated with the invasive treatment have become issues. On the other hand, for the atrophic age-related macular degeneration, an effective therapy has not been established as of this movement. Establishing a more non-invasive therapy for the exudative age-related macular degeneration and establishing an effective therapy for the atrophic age-related macular degeneration have been desired. The hereditary retinal degeneration includes a macular dystrophy such as a pigmentary degeneration of retina and Stargardt, cone-rod dystrophy, amaurosis congenita of Leber, and the like. Currently, while gene replacement therapy has been tried for some of the hereditary retinal degeneration, no permanent cure is available for most of the hereditary retinal degeneration, and establishing a new medical treatment has been desired.

Inventors of the present invention, recently, have submitted an interesting report about effects of violet light on eyes, and in Patent Document 1 and Non-Patent Document 1, for instance, it has been proposed that light of a specific wavelength region is effective in preventing myopia and suppressing myopia, and with myopic population still on the rise globally in recent years, high expectations are placed. Effects of light on a human body have been studied from various perspectives in recent years, and reported on the basis of new findings. For instance, facts such as, circadian rhythm is improved by bathing in the sunlight (Non-Patent Document 2), and light irradiated from a display such as a liquid-crystal display in which LED (light emitting diode) lighting and LED is used as a backlight has a substantial effect on body and mind (Non-Patent Document 3) have been reported.

### Citation List

### Non-patent Documents

Non-Patent Document 1: Hidemasa Torii et al., EBioMedicine, 'DOI: http://dx.doi.org/10.1016/j.ebiom.2016.12.007'
Non-Patent Document 2: Anti-aging Medicine by Megumi Hatori and Kazuo Tsubota, Japanese Society of Anti-aging Medicine Magazine Vol. 11, No. 3, 065 (385) - 072 (392), (2015)
Non-Patent Document 3: Blue Light Hazard by Kazuo Tsubota, Published by Shueisha on November 20, 2013

### Patent Documents

Patent Document 1: WO2015/186723A1

### Summary of the Invention

### Problems to be Solved by the Invention

One of the objectives of the present disclosure is to provide a new method for treating a retinal degeneration such as an age-related macular degeneration and a hereditary retinal degeneration, and an apparatus used for the same. The object of the present disclosure includes providing a method for treating the retinal degeneration such as the age-related macular degeneration and the hereditary retinal degeneration, which does not involve an invasive treatment (intravitreal injection) and complications (such as intraocular inflammation) and side effects (such as cerebral infarction) associated with invasive treatment. Particularly, one of the objectives of the present disclosure is to provide a method for treating an atrophic age-related macular degeneration and the hereditary retinal degeneration for which, an effective therapy is not yet available.

### Means for Solving the Problems

The inventors of the present invention discovered that by irradiating a subject (for example, eyes) with light of a specific wavelength region, it is possible to treat and/or prevent a retinal degeneration such as an age-related macular degeneration and a hereditary retinal degeneration, and accomplished the present invention. In other words, the present disclosure provides a method for treating and/or preventing the retinal degeneration such as the age-related macular degeneration and hereditary retinal degeneration by irradiating a subject with light of a specific wavelength region. Preferably, the present disclosure provides the method for treating and/or preventing the retinal degeneration such as the age-related macular degeneration and the hereditary retinal degeneration by irradiating the subject with violet light (VL) continuously or in pulses at a specific wavelength.

Moreover, according to one aspect, the present disclosure provides the method for treating and/or preventing the retinal degeneration such as the age-related macular degeneration and the hereditary retinal degeneration by irradiating light of a specific wavelength region by controlling a light irradiating apparatus. In some embodiments, the light irradiating apparatus includes a light source which is capable of irradiating a living body with light of a specific wavelength region continuously or at pulsed at a specific frequency and a controller which controls irradiation of the light source, such as controlling the blinking frequency. This includes a method for treating and/or preventing symptoms of the retinal degeneration such as the age-related macular degeneration and the hereditary retinal degeneration in the subject by irradiating the subject with light of a specific wavelength region by using the light irradiating apparatus.

According to another aspect, the present disclosure is related to a method for treating and/or preventing symptoms of a retinal degeneration such as an age-related macular degeneration and a hereditary retinal degeneration in a subject requiring treatment and/or prophylaxis, and the method includes irradiating the subject with light of a specific wavelength region. The subject for which the method according to the present disclosure is applicable may be a patient of the retinal degeneration such as the age-related macular degeneration and the hereditary retinal degeneration.

According to still another aspect, the present disclosure provides a method for treating and/or preventing symptoms associated with the retinal degeneration such as the age-related macular degeneration and the hereditary retinal degeneration in particular.

According to still another aspect, the present disclosure provides a component which is built-in or attached to an apparatus or an instrument, and which is replaceable.

According to still another aspect, the present disclosure provides a method or a system for replacing, repairing, or carrying out maintenance of the apparatus, the instrument, or the component.

In other words, the present disclosure is as follows.

### [Item 1]

A method for treating and/or preventing retinal degeneration, comprising:
irradiating a subject with light of a specific wavelength region by using a light irradiating apparatus.

### [Item 2]

The method according to item 1, wherein the retinal degeneration is either age-related macular degeneration or hereditary retinal degeneration.

### [Item 3]

The method according to item 2, wherein the age-related macular degeneration is either exudative age-related macular degeneration or atrophic age-related macular degeneration.

### [Item 4]

The method according to item 2, wherein the age-related macular degeneration is associated with at least one symptom selected from a group consisting of low vision, metamorphopsia, central scotoma, and complete loss of vision.

### [Item 5]

The method according to item 4, wherein at least one symptom selected from a group consisting of low vision, metamorphopsia, central scotoma, and complete loss of vision is caused by age-related macular degeneration.

### [Item 6]

The method according to item 2, wherein an element of the age-related macular degeneration is at least one selected from a group consisting of neovascular volume, amount of subretinal fluid, amount of intraretinal edema, geographic atrophy range, and range of photoreceptor cell layer loss.

### [Item 7]

The method according to item 2, wherein the age-related macular degeneration is either a precursor lesion or a progressive age-related macular degeneration.

### [Item 8]

The method according to item 1, wherein the light irradiating apparatus includes a light source which is capable of irradiating the subject with light of a specific wavelength region continuously or pulsed at a specific frequency, and a controller which controls irradiation of the light source.

### [Item 9]

The method according to item 1, wherein the subject is either undergoing a or has undergone treatment and/or prophylaxis of retinal degeneration.

### [Item 10]

The method according to item 1, wherein the specific wavelength region includes a wavelength range of 360 nm to 400 nm.

### [Item 11]

The method according to item 1, wherein the light is made to pulse at a blinking frequency in a range of 30 Hz to 70 Hz.

### [Item 12]

The method according to item 1, wherein the light is irradiated during daytime.

### [Item 13]

The method according to item 1, wherein the light is irradiated for one hour or more.

### [Item 14]

The method according to item 1, wherein the light is irradiated such that an irradiance of light incident on eyes of the subject is in a range of 0.5 µW/cm² to 1000 µW/cm².

### [Item 15]

The method according to item 1, wherein the light irradiating apparatus is spectacles with a light source or a spectacle frame with a light source, a desktop light source, a mobile terminal mounted light source, a light source installed nearby or in front of face, a portable light source, an indoor lighting, or a desk lamp.

### [Item 16]

An apparatus for treating and/or preventing a retinal degeneration by irradiating a subject with light of a specific wavelength region.

### [Item 17]

The apparatus according to item 16, wherein the apparatus irradiates light of a specific wavelength region continuously or at a blinking frequency.

### [Item 18]

The apparatus according to item 16, comprising:
a controller which controls emission of light irradiated.

### [Item 19]

The apparatus according to item 18, wherein the controller, by transceiving to and from an isolated controller such as a portable terminal, carries out control by changing at least any one irradiation condition selected from a group of irradiation conditions consisting of blinking frequency of the light of a specific wavelength region, irradiance, irradiation time, irradiation-start time, and irradiation-end time.

### [Item 20]

The apparatus according to item 16, comprising:
a light source; and
a driving circuit which drives the light source.

### [Item 21]

The apparatus according to item 20, wherein the driving circuit includes at least one processor which is communicably connected to the light source and at least one memory, and at least one memory for storing commands executable by the processor.

### [Item 22]

The apparatus according to item 16, wherein the retinal degeneration is either age-related macular degeneration or hereditary retinal degeneration.

### [Item 23]

The apparatus according to item 22, wherein the apparatus treats and/or prevents the age-related macular degeneration which is either an exudative age-related macular degeneration or an atrophic age-related macular degeneration.

### [Item 24]

The apparatus according to item 22, wherein the age-related macular degeneration is associated with at least one symptom selected from a group consisting of low vision, metamorphopsia, centra scotoma, and complete loss of vision.

### [Item 25]

The apparatus according to item 24, wherein at least one symptom selected from a group consisting of low vision, metamorphopsia, central scotoma, and complete loss of vision is caused by age-related macular degeneration.

### [Item 26]

The apparatus according to item 22, wherein an element of the age-related macular degeneration is at least one selected from a group consisting of neovascular volume, amount of subretinal fluid, amount of intraretinal edema, geographic atrophy range, and range of photoreceptor cell layer loss.

### [Item 27]

The apparatus according to item 22, wherein the age-related macular degeneration is either precursor lesion or progressive age-related macular degeneration.

### [Item 28]

The apparatus according to item 16, wherein the subject is either undergoing or has undergone treatment and/or prophylaxis for the retinal degeneration.

### [Item 29]

The apparatus according to item 16, wherein the specific wavelength region includes a wavelength range of 360 nm to 400 nm.

### [Item 30]

The apparatus according to item 17, wherein the blinking frequency is in a range of 30 Hz to 70 Hz.

### [Item 31]

The apparatus according to item 16, wherein the light is irradiated during daytime.

### [Item 32]

The apparatus according to item 16, wherein the light is irradiated for one hour or more.

### [Item 33]

The apparatus according to item 16, wherein the light is irradiated such that an irradiance of light incident on eyes of a subject is in a range of 0.5 µW/cm² to 1000 µW/cm².

### [Item 34]

The apparatus according to item 16, wherein the apparatus is spectacles with a light source or a spectacle frame with a light source, a desktop light source, a mobile terminal mounted light source, a light source installed nearby or in front of face, a portable light source, an indoor lighting, or a desk lamp.

### [Item 35]

The apparatus according to item 16, wherein light of other wavelength, sound, vibrations, magnetic field, or electric field is applied in addition, together with irradiation of light of a specific wavelength region.

### [Item 36]

A computer-readable medium which is a non-transitory computer-readable medium having commands stored therein, and which is capable of causing an apparatus according to any one of claims 16 to 35, including a light source which is capable of irradiating a living body with light of a specific wavelength region continuously or pulsed at a specific frequency and a controller which controls the blinking frequency of the light source, to perform the following steps when a command is executed by a processor:
a step of operating the apparatus such that the controller controls the blinking frequency of the light source to 0 Hz or to a frequency in a range of 30 Hz to 75 Hz; and
a step of operating the apparatus such that the light source irradiates a living body with light of a wavelength in a range of 360 nm to 400 nm.

### [Item 37]

An instrument for treatment and/or prophylaxis of retinal degeneration by light stimulation, comprising:
a glass, a spectacle lens, or a contact lens which allows violet light to pass through.

### [Item 38]

A component which is built-in or attached to an apparatus according to item 16, and which is replaceable.

### [Item 39]

A component which is built-in or attached to an instrument according to item 37, and which is replaceable.

### [Item 40]

A system which replaces, repairs, or carries out maintenance of an apparatus according to item 16.

### [Item 41]

A system which replaces, repairs, or carries out maintenance of an instrument according to item 37.

### [Item 42]

A system which replaces, repairs, or carries out maintenance of a component according to item 38 or item 39.

### [Item 43]

A method for replacing, repairing, or carrying out maintenance of an apparatus according to item 16.

### [Item 44]

A method for replacing, repairing, or carrying out maintenance of an instrument according to item 37.

### [Item 45]

A method for replacing, repairing, or carrying out maintenance of a component according to item 38 or item 39.

Apart from the abovementioned items, the present disclosure also includes the following content related to the method.
- the method for treating and/or preventing the retinal degeneration which includes irradiating the subject with light of a specific wavelength region by using a light irradiating apparatus.
- the method, wherein the light is violet light.
- the method, wherein the specific wavelength region includes a wavelength of approximately 380 nm.
- the method, wherein the blinking frequency is a frequency in a range of 35 Hz to 60 Hz.
- the method, wherein the blinking frequency is 40 Hz.
- the method, wherein the light is irradiated continuously for one hour or more.
- the method, wherein the light is irradiated continuously for two hours or more.
- the method, wherein the subject is a human.

Moreover, the present disclosure also includes the following content related to the apparatus.
- the apparatus for treatment and/or prophylaxis of the retinal degeneration which treats and/or prevents the retinal degeneration by irradiating a subject with light of a specific wavelength region continuously or pulsed at a specific frequency, and has a light source which irradiates the light of a specific wavelength region continuously or pulsed at a specific frequency.
- the apparatus, wherein the light is violet light.
- the apparatus, wherein the specific wavelength region includes a wavelength of approximately 380 nm.
- the apparatus, wherein the blinking frequency is in a range of 35 Hz to 60 Hz.
- the apparatus, wherein the blinking frequency is 40 Hz.
- the apparatus, wherein the light is irradiated continuously for one hour or more.
- the apparatus, wherein the light is irradiated such that an irradiance of light incident on eyes of a subject is in a range of 0.5 µW/cm² to 1000 µW/cm².
- the apparatus wherein the light is irradiated continuously for one hour or more.

### Brief Description of the Drawings

Fig. 1 is an example of violet light spectacles configured to irradiate violet light;
Fig. 2 is a graph showing a relationship of wavelength and spectral irradiance of violet fluorescent light;
Fig. 3 is a light spectrum of an LED with a peak wavelength of 375 nm;
Fig. 4 is a block diagram of an embodiment of an apparatus for controlling biological function according to the present invention;
Fig. 5 is a graph showing a volume of choroidal neovascular (CNV) measured for a control group and a violet light group in a test (examination) using a mouse choroidal neovascular model;
Fig. 6 is a result of electroretinogram measurement for each irradiation intensity in a test using a mouse photodamage model; and
Fig. 7 is a result of measurement of a thickness of outer nuclear layer (ONL) of retina and a length of photoreceptor outer segment (OS) in a test using a mouse photodamage model.

### Mode for Carrying Out the Invention

The retinal degeneration to be treated and/or prevented according to the present disclosure includes the age-related macular degeneration and the hereditary retinal degeneration. The hereditary retinal degeneration includes a macular dystrophy such as pigmentary degeneration of retina and Stargardt, cone-rod dystrophy, and amaurosis congenita of Leber.

The age-related macular degeneration to be treated and/or prevented according to the present disclosure is a disease in which an abnormal degeneration occurs in a site called as macula of a central part of the retina with aging, and visual function (visual power, visual field) is declined. The age-related macular degeneration to be treated and/or prevented according to the present disclosure includes the exudative age-related macular degeneration and the atrophic age-related macular degeneration. In the exudative age-related macular degeneration, a neovascular vessel is developed in choroid coat under macula, and grows toward retina. The neovascular vessel is an abnormal blood vessel which does not exist in a normal retina, and a blood vessel wall being weak, leakage of blood component and bleeding are susceptible to occur, and these exudates impair macular function and cause vision disorder. Whereas, the atrophic age-related macular degeneration is a phenomenon in which macular tissue (retinal pigment epithelium and photoreceptor cell) undergoes atrophic degeneration with aging.

Symptoms of the exudative age-related macular degeneration to be treated and/or prevented by the present invention includes specifically, symptoms such as low vision, metamorphopsia, central scotoma, and complete loss of vision. Central scotoma is a symptom of an advanced exudative age-related macular degeneration. Complete loss of vision is a symptom which may occur when the neovascular vessel become uncontrollable.

Symptoms of the atrophic age-related macular degeneration to be treated and/or prevented by the present invention include specifically, symptoms such as low vision, metamorphopsia, and central scotoma. Central scotoma is a symptom of an advanced age-related macular degeneration.

Elements of the age-related macular degeneration to be treated and/or prevented by the present invention include specifically, elements such as neovascular volume, amount of subretinal fluid, amount of intraretinal edema, geographic atrophy range, and range of photoreceptor cell layer loss.

In the context of the present disclosure, patients of the age-related macular degeneration, in the classification of the degree of severity, are classified as a precursor lesion such as drusen, an exudative type, and an atrophic type progressive age-related macular degeneration, and may include any of the three.

When applying the method according to the present disclosure, the subject may be a patient who is undergoing currently or has undergone in the past the treatment and/or prophylaxis for at least one of the retinal degeneration such as the age-related macular degeneration and the hereditary retinal degeneration. The therapy for the exudative age-related macular degeneration includes photodynamic therapy (PDT) in which, laser light is irradiated to a lesion after administering a photosensitizer (VISUDYNE) into the body. Moreover, the treatment also includes antibody therapy for VEGF (vascular endothelial growth factor). Apart from this, as a treatment for the age-related macular degeneration, a therapy of direct laser photocoagulation and a therapy of decannulating neovascular vessel surgically were performed in the past, but are not performed currently.

When applying the method according to the present disclosure, the subject may be a patient who is being administered currently or was administered in the past at least one anti-retinal degeneration agent such as anti-age-related macular degeneration agent.

The anti-age-related macular degeneration agents include, apart from anti VEGF (vascular endothelial growth factor) antibody which suppresses the growth of the neovascular vessel, VEGF and angiopoietin 2 simultaneous inhibitory anti body. Vascular endothelial growth factor (VEGF) anti body, for instance, is administered by intravitreal injection.

Moreover, according to another aspect, the present disclosure is related to the method for treating and/or preventing the retinal degeneration, which includes irradiating the subject with light of a specific wavelength region by using a light irradiating apparatus. More specifically, the present disclosure is related to the method for treating and/or preventing the retinal degeneration such as the age-related macular degeneration and the hereditary retinal degeneration for the subject requiring treatment and/or prophylaxis, which is a method for treating and/or preventing the retinal degeneration such as the age-related macular degeneration and the hereditary retinal degeneration, which includes irradiating the subject with light of a specific wavelength region by using the light irradiating apparatus.

Moreover, according to another aspect, the present disclosure is related to a method for treating and/or preventing a choroidal neovascularization in a patient suffering from the age-related macular degeneration requiring treatment and/or prophylaxis, which includes irradiating light of a specific wavelength region to the patient. Moreover, in some embodiments, the present disclosure is related to the method for treating and/or preventing the age-related macular degeneration in the subject requiring treatment and/or prophylaxis which includes treating and/or preventing the age-related macular degeneration in the subject by treating and/or preventing the choroidal neovascularization in the subject by irradiating the subject with light of a specific wavelength region. It has been known that in the age-related macular degeneration, the choroidal neovascularization is developed in a choroid coat under macula of a central portion of the retina, and treatment and/or prophylaxis of the choroidal neovascularization is considered to be vital for the treatment and/or prophylaxis of the age-related macular degeneration.

In some embodiments, the light irradiating apparatus includes a light source which is capable of irradiating a subject with light of a specific wavelength region continuously or pulsed at a specific frequency, and a controller which controls the irradiation of the light source.

In some embodiments, the light used is violet light.

In some embodiments, the specific wavelength region used includes a wavelength range of 360 nm to 400 nm, and particularly includes a wavelength of approximately 380 nm.

In some embodiments, the blinking frequency used is either 0 Hz or a frequency in a range of 30 Hz to 70Hz, and in particular, 0 Hz or a frequency in a range of 35 Hz to 60 Hz, and more specifically, 0 Hz or a frequency of approximately 40 Hz for example.

In some embodiments, irradiation conditions further include an irradiation time of the light source. More specifically, irradiation time includes irradiating for one hour or more.

In some embodiments, the light is irradiated such that an irradiance of light incident on eyes of a subject is in a range of 0.5 µW/cm² to 1000 µW/cm².

In some embodiments, the light source may be spectacles with a light source or a spectacle frame with a light source, a desktop light source, a mobile terminal mounted light source, a light source installed nearby or in front of face, a portable light source, an indoor lighting, or a desk lamp.

The light sources include more preferably, spectacles with a light source or a spectacle frame with a light source, a desktop light source, a mobile terminal mounted light source, a light source installed nearby or in front of face, a portable light source, or a desk lamp.

The light sources include even more preferably, spectacles with a light source or a spectacle frame with a light source, a desktop light source, a mobile terminal mounted light source, or a light source installed nearby or in front of face.

The light sources include even more preferably, spectacles with a light source or a spectacle frame with a light source, or a desktop light source.

The light sources include most preferably, spectacles with a light source or a spectacle frame with a light source.

In some embodiments, the subject for which the method according to the present disclosure is applied is a human.

According to one aspect, the present disclosure is related to an apparatus which treats and/or prevents the retinal degeneration by irradiating the subject with light of a specific wavelength region. More specifically, the present disclosure is related to the apparatus for treatment and/or prophylaxis of the retinal degeneration such as the age-related macular degeneration and the hereditary retinal degeneration by light stimulation, having at least one light source which emits light and a driving circuit which drives the light source, and the light emitted by the light source is a light of a specific wavelength region which produces an effect of treatment and/or prophylaxis of the retinal degeneration such as the age-related macular degeneration and the hereditary retinal degeneration by being irradiated to a living body.

Moreover, according to one aspect, the present disclosure is related to an apparatus for treatment and/or prophylaxis of the symptoms and/or other elements having at least one light source which emits light and a driving circuit which drives the light source, by irradiating a living body with light emitted by the light source.

In some embodiments, the driving circuit in the apparatus according to the present disclosure includes at least one processor which is communicably connected to the light source and at least one memory, and at least one memory for storing command executable by the processor.

According to one aspect, the present disclosure is related to a method of operating the apparatus according to the present disclosure which includes a light source which is capable of irradiating a living body with light of a specific wavelength region continuously or pulsed at a specific frequency and a controller which controls irradiation of the light source, such as controlling the blinking frequency, which includes a step of controlling the blinking frequency of the light source to 0 Hz or to a frequency in a range of 30 Hz to 75 Hz, and a step of irradiating a living body with light of a wavelength in a range of 360 nm to 400 nm by the light source.

According to one aspect, the present disclosure is related to a computer program which causes an apparatus including a light source which is capable of irradiating a living body with light of a specific wavelength region continuously or pulsed at a specific frequency and a controller which controls the irradiation of the light source, such as controlling the blinking frequency, to perform a method of operating according to the present disclosure.

**In** the method and apparatus according to the present disclosure, the light is violet light for example. According to the present invention, since it is possible to irradiate the living body with violet light of a wavelength outside the visible light region it is possible to have an effect on the living body without feeling flickering or dazzling as in a case of white light. Moreover, the violet light is light of a wavelength in a range of 360 nm to 400 nm, and light of this wavelength has a low visible sensitivity as compared to the visible sensitivity of white light, and is light of a wavelength region which imparts no uncomfortable feeling or hardly any uncomfortable feeling to a living body (particularly a human). Moreover, in the method and apparatus according to the present disclosure, it is preferable to irradiate light during daytime.

In some embodiments of the present disclosure, light of a wavelength in a range of 350 nm to 400 nm, such as light of one of wavelengths 350 nm, 360 nm, 370 nm, 380 nm, 390 nm, or 400 nm, or light of an arbitrary wavelength included in a range stipulated by the arbitrary wavelength mentioned above (for example, in a range of 370 nm to 390 nm) may be used. In some embodiments of the present disclosure, a wavelength of approximately 380 nm is included. Moreover, the term 'approximately' used in the present specification indicates that it includes a value that is within a variation of up to ±5% of the value modified by this term.

In the method and apparatus according to the present disclosure, the condition for irradiation of light is that the light is irradiated continuously (in other words, 0 Hz) or irradiated at a blinking frequency in a range of higher than 0 Hz up to 150 Hz.

According to some embodiments of the present disclosure, for the light which is irradiated continuously (0 Hz) or the light having a blinking frequency in the range of 30 Hz to 75 Hz, such as the light having any of the blinking frequencies 30 Hz, 35 Hz, 40 Hz, 45 Hz, 50 Hz, 55 Hz, 60 Hz, 65 Hz, 70 Hz, or 75 Hz, or light of an arbitrary blinking frequency included in a range stipulated by the abovementioned arbitrary blinking frequencies (for example, in a range of 35 Hz to 45 Hz) may be used. In some embodiments of the present disclosure, the blinking frequency is approximately 40 Hz.

In the method and apparatus according to the present disclosure, the light can be irradiated such that the irradiance of light incident on eyes of a subject is in a range of 0.5 µW/cm² to 1000 µW/cm² or in a range of 0.1 µW/cm² (0.001 W/m²) to 5000 µW/cm² (50 W/m²). Moreover, the irradiance of light incident on eyes of the subject may be in a range of 100 µW/cm² to 1000 µW/cm². According to some embodiments, by irradiating light such as violet light in the abovementioned range of irradiance, it is possible to have an effect on macula. Even though the violet light in general, is a weak light of an extremely small amount (light with a weak optical sensitivity) in particular, it has been verified that a characteristic phenomenon may occur.

In the method and apparatus according to the present disclosure, the controller of the apparatus, by transceiving to and from an isolated controller such as a portable terminal is capable of carrying out control by changing the irradiation conditions of light (including irradiating continuously or at a blinking frequency) such as irradiance, irradiation time, irradiation-start time, irradiation-end time, and irradiating continuously or at a blinking frequency. According to some embodiments, the various irradiation conditions mentioned above being subjected to isolated control, it is possible to set arbitrarily irradiation conditions appropriate for producing an effect of treatment and/or prophylaxis of the symptoms, or in other words, at least one symptom selected from a group consisting of low vision, metamorphopsia, central scotoma, and complete loss of vision.

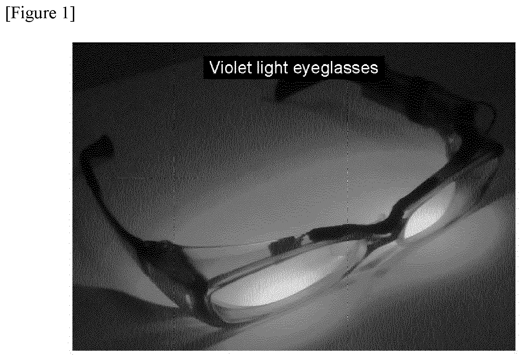

In the method and apparatus according to the present disclosure, the light source may be a light source installed nearby or in front of face, such as spectacles with a light source (for example, refer to Fig. 1) or a spectacle frame with a light source, a desktop light source, and a mobile terminal mounted light source. According to some embodiments, as it is possible to irradiate specific light from the light source installed nearby or in front of face such as spectacles with a light source or a spectacle frame with a light source that can be worn easily and has no uncomfortable feeling in daily use, they are highly practical, and are capable or irradiating light any time even in various situations and varied environments.

In the method and apparatus according to the present disclosure, the light source may be a non-mounting type light source such as a portable light source, or may be a mounting type light source such as an indoor lighting, a desk lamp, and a dedicated device. According to some embodiments, it is possible to make it an apparatus in various forms of light source appropriate for an environment of usage. For instance, the light source may be used in combination with a glass, a spectacle lens, or a contact lens that allows violet light to pass through. Moreover, sunlight that has passed through a glass, a spectacle lens, or a contact lens that allows violet light to pass through, may be used as the light source.

A method by light stimulation according to the present disclosure is a method which treats and/or prevents the retinal degeneration such as the age-related macular degeneration and the hereditary retinal degeneration by irradiating the living body with light of a specific wavelength region continuously or pulsed at a specific frequency (for example, mammals including human), and is characterized by controlling emission of light by which a symptom in the living body having received the light, in other words, at least one symptom selected from a group consisting of low vision, metamorphopsia, central scotoma, and complete loss of vision is improved or suppressed.

The apparatus according to the present disclosure is an apparatus for treating and/or preventing the retinal degeneration such as the age-related macular degeneration and the hereditary retinal degeneration by irradiating the living body with violet light continuously, and is characterized by including a light source which emits the violet light and a light-emission time controller which makes irradiate the violet light at a specific time or for a specific period of time.

The present disclosure includes a component which is built-in or attached to an apparatus or an instrument, and which is replaceable. For instance, it is preferable that the apparatus or the instrument of the present disclosure is maintained at a certain degree of quality all the time in order to be able to carry out treatment and/or prophylaxis of the retinal degeneration, and from a viewpoint of maintaining the quality, it is preferable to carry out the necessary replacement of components, and more preferably, to carry out the necessary replacement of the components periodically. These components include components such as the light source, the controller which controls the emission of light irradiated, peripheral equipment, and battery. More specifically, the components include components of which the performance and functioning are depleted with time, and include for example, components such as an LED (light emitting diode) element as a light source, driving circuit, memory, and lithium secondary battery as a battery.

Moreover, the present disclosure includes a maintenance method or a maintenance system for replacing, repairing or carrying out maintenance of the apparatus, the instrument, or the components that are replaceable. As mentioned above, it is preferable to maintain the apparatus or the instrument according to the present disclosure at a certain degree of quality all the time such that the treatment and/or prophylaxis of the retinal degeneration can be carried out favorably. Therefore, it is preferable to carry out maintenance (checking of the apparatus or instrument), replacement of components, or repair, and more preferably, the regular maintenance (checking of the apparatus or instrument), regular replacement of components, or regular repair. The present disclosure includes a method for such maintenance, component replacement, and repair, and a system for carrying out the same, and more preferably, includes a method for regular maintenance, component replacement, and repair, and a system for carrying out the same. Such method includes a method in which sensors which detect state of each component in the apparatus or the instrument, and carries out the maintenance, component replacement, and repair according to result of detection by these sensors.

Furthermore, as it will be described below while referring to diagrams, in some embodiments of the present disclosure, a method for treating and/or preventing the retinal degeneration such as the age-related macular degeneration and the hereditary retinal degeneration in the subject requiring treatment and/or prophylaxis, the method for treating and/or preventing the symptom, or in other words, at least one symptom selected from the group consisting of low vision, metamorphopsia, central scotoma, and complete loss of vision in the subject requiring treatment and/or prophylaxis, the apparatus for treatment and/or prophylaxis of the retinal degeneration such as the age-related macular degeneration and the hereditary retinal degeneration by light stimulation, the apparatus from treatment and/or prophylaxis of the symptom, or in other words, at least one symptom selected from the group consisting of low vision, metamorphopsia, central scotoma, and complete loss of vision by light stimulation, the method of operating the apparatus, and a computer program which makes execute the method of operating are provided.

The method for treating and/or preventing the retinal degeneration such as the age-related macular degeneration and the hereditary retinal degeneration in the subject requiring treatment and/or prophylaxis, the method for treating and/or preventing the symptoms, or in other words, at least one symptom selected from a group consisting of low vision, metamorphopsia, central scotoma, and complete loss of vision in a subject requiring treatment and/or prophylaxis, the apparatus for treatment and/or prophylaxis of the retinal degeneration such as the age-related macular degeneration and the hereditary retinal degeneration by light stimulation, the apparatus for treatment and/or prophylaxis of the symptoms, or in other words, at least one symptom selected from the group consisting of low vision, metamorphopsia, central scotoma, and complete loss of vision, the method of operating the apparatus, and the computer program which makes execute the method of operating according to the present disclosure will be described below while referring to diagrams. The present invention is not restricted to the following embodiments and example, and includes various modified examples and application examples which fall within the scope of the present invention.

### [Method for treating and/or preventing the retinal degeneration such as the age-related macular degeneration and the hereditary retinal degeneration]

The method for treating and/or preventing the retinal degeneration such as the age-related macular degeneration and the hereditary retinal degeneration according to the present disclosure includes irradiating the subject requiring treatment and/or prophylaxis with light of a specific wavelength region.

Inventors of the present invention discovered that by irradiating the subject with light of a specific wavelength region, it is possible to treat and/or prevent the retinal degeneration such as the age-related macular degeneration and the hereditary retinal degeneration as indicated by a decrease in CNV (choroidal neovascularization) volume. Therefore, one aspect of the present disclosure is related to the method for treating and/or preventing the retinal degeneration such as the age-related macular degeneration and the hereditary retinal degeneration in the subject requiring treatment and/or prophylaxis, which includes irradiating the subject with light of a specific wavelength region. In some embodiments, by irradiating the subject with light of a specific wavelength region, CNV (choroidal neovascularization) is suppressed. Therefore, one aspect of the present disclosure is related to a method for suppressing CNV (choroidal neovascularization) in the subject requiring treatment and/or prophylaxis, which includes irradiating the subject with light of a specific wavelength region. Here, the subject may be a human suffering from the retinal degeneration such as the age-related macular degeneration and the hereditary retinal degeneration, or a human at risk of developing the retinal degeneration such as the age-related macular degeneration and the hereditary retinal degeneration.

As described heretofore, the symptoms of the age-related macular degeneration include low vision, metamorphopsia, central scotoma, and complete loss of vision.

In diagnosis of the age-related macular degeneration, examination of the ocular fundus, optical coherence tomography (OCT), and fundus fluorescein angiography are combined, and a comprehensive judgment is made to differentiate whether it is age-related macular degeneration or a disorder other than age-related macular degeneration.

Patients of the age-related macular degeneration, in the classification of the degree of severity, may be classified as a precursor lesion such as drusen, an exudative type, and an atrophic type progressive age-related macular degeneration. Moreover, the evaluation of the age-related macular degeneration is made by judging comprehensively upon combining examination of ocular fundus, optical coherence tomography (OCT), and fundus fluorescein angiography, and optical coherence tomography (OCT) being minimally invasive and quantitatively superior, is used frequently.

As mentioned above, one aspect of the present disclosure is related to the method for treating and/or preventing the retinal degeneration such as the age-related macular degeneration and the hereditary retinal degeneration in the subject requiring treatment and/or prophylaxis, which includes irradiating the subject with light of a specific wavelength region. The method may include improving or preventing the symptoms in the subject, or in other words, at least one symptom selected from the group consisting of low vision, metamorphopsia, central scotoma, and complete loss of vision, by irradiating the subject with light of a specific wavelength region, and treating and/or preventing the retinal degeneration such as the age-related macular degeneration and the hereditary retinal degeneration in the subject.

The treatment for the age-related macular degeneration, currently, for the exudative age-related macular degeneration is mainly intravitreal injection of an anti-vascular endothelial growth factor (VEGF) anti-body. As a medicinal agent, apart from anti-VEGF anti-body (antiangiogenic agent) which suppresses the growth of neovascular vessel, an agent such as VEGF and angiopoietin 2 simultaneous inhibitory anti body is used. Therefore, the method for treatment and/or prophylaxis according to the present disclosure may be performed on a patient who is being administered or a patient who was administered these medicinal agents. The method for treatment and/or prophylaxis according to the present disclosure may be performed in combination with the administration of these medicinal agents.

In a patient of the age-related macular degeneration, it has been revealed that CNV (choroidal neovascularization) is observed in the choroid coat under macula of a central portion of the retina. The inventors of the present invention discovered that by irradiating the subject with light of a specific wavelength region, it is possible to suppress CNV (choroidal neovascularization) of the choroid coat under the macula of the central portion of the retina. Therefore, one aspect of the present disclosure is related to a method for suppressing or improving CNV (choroidal neovascularization) in the choroidal coat under macula of the central portion of the retina of a patient suffering from the age-related macular degeneration, which includes irradiating the subject with light of a specific wavelength region.

In some embodiments according to the present disclosure, the light used may be violet light. Moreover, in some embodiments of the method according to the present disclosure, the specific wavelength used includes a wavelength in a range of 360 nm to 400 nm, and can particularly include a wavelength of approximately 380 nm. The blinking frequency for instance, may be 0 Hz or a frequency in a range of 30 Hz to 70 Hz, and particularly, may be 0 Hz or a frequency in a range of 35 Hz to 60 Hz. The blinking frequency in particular, may be 0 Hz or approximately 40 Hz. The irradiation conditions may further include an irradiation time of the light source. The light source may be spectacles with a light source or a spectacle frame with a light source, a desktop light source, for example, a desktop light and a reading light kept by bed side, a desk lighting, a mobile terminal mounted light source, a light source installed nearby or in front of face, a portable light source, an indoor lighting, a wall-mounted lighting, a ceiling light, or a desk lamp, but is not restricted to these light sources.

In some embodiments of the present disclosure, the specific time of irradiating light is an arbitrary time in a range of 10 seconds to 24 hours per day, and any time such as 10 seconds, 30 seconds, 45 seconds, one minute, three minutes, five minutes, 10 minutes, 15 minutes, 30 minutes, 45 minutes, one hour, two hours, three hours, four hours, five hours, six hours, seven hours, eight hours, nine hours, 10 hours, 11 hours, 12 hours, 18 hours, and 24 hours, or may be any arbitrary time in a range stipulated by the abovementioned arbitrary time (for example, a range of one hour to 12 hours). A specific period of irradiating the light continuously may be for example, one day, two days, three days, four days, five days, six days, one week, two weeks, three weeks, one month, two months, three months, six months, one year, two years, three years, or a period more than that.

In some embodiments of the present disclosure, light such as light of a fluorescent lamp may be irradiated in addition to irradiating the light of a specific wavelength region. Color temperature of light irradiated by a lamp such as fluorescent lamp is in a range of 2600 Kelvin to 7100 Kelvin for example, and in a range of 4600 Kelvin to 5500 Kelvin for example. A time slot of irradiating light by a lamp such as fluorescent lamp is for example, 7 am to 9 pm, 8 am to 8 pm, and 9 am to 7 pm. Time of irradiating light by a lamp such as fluorescent lamp is for example, nine hours to 15 hours per day, 10 hours to 14 hours per day, and 11 hours to 13 hours per day. Time of irradiating any of the light of a specific wavelength region and the light of a fluorescent lamp is for example one hour to five hours per day and two hours to four hours per day. Each day, the light from a lamp such as fluorescent lamp may be irradiated before irradiating the light of a specific wavelength region per day. Time of irradiating the light by a lamp such as fluorescent lamp before irradiating the light of a specific wavelength region per day is for example, six hours to 12 hours, seven hours to 11 hours, and eight hours to 10 hours per day. Each day, the light of a lamp such as fluorescent lamp may be irradiated even after finishing irradiation of the light of a specific wavelength region per day, and the light of a lamp such as fluorescent lamp may not be irradiated after finishing irradiation of the light of a specific wavelength region per day. Time of irradiating the light by a lamp such as fluorescent lamp after finishing irradiation of the light of a specific wavelength region per day is for example, 0 minutes to one hour, 0 minutes to 30 minutes, and 0 minutes to 15 minutes.

One aspect of the present disclosure is related to the method for treating and/or preventing the retinal degeneration such as the age-related macular degeneration and the hereditary retinal degeneration in the subject requiring treatment and/or prophylaxis, which includes irradiating the subject with light of a specific wavelength region by controlling the light irradiating apparatus, and accordingly, improving or suppressing at least one symptom selected from the group consisting of low vision, metamorphopsia, central scotoma, and complete loss of vision, thereby treating and/or preventing the retinal degeneration such as the age-related macular degeneration and the hereditary retinal degeneration in the subject. In some embodiments, the light irradiating apparatus is capable of irradiating violet light. Moreover, in some embodiments, the light irradiating apparatus is capable of irradiating light of a wavelength in a range of 360 nm to 400 nm, and particularly, light of a wavelength of approximately 380 nm. The light irradiating apparatus may be capable of controlling the blinking frequency to 0 Hz or to a frequency in a range of 30 Hz to 70 Hz for example, and particularly to 0 Hz or to a frequency in a range of 35 Hz to 60 Hz, and the blinking frequency may be 0 Hz or approximately 40 Hz in particular. The light irradiating apparatus may be an apparatus for which the irradiation time is controllable. The light source may be in the form of spectacles with a light source or a spectacle frame with a light source, a desktop light source, a mobile terminal mounted light source, a light source installed nearby or in front of face, a portable light source, an indoor lighting, or a desk lamp, but is not restricted to these forms.

### [Apparatus]

One aspect of the present disclosure is related to the apparatus which treats and/or prevents the retinal degeneration by irradiating the subject with light of a specific wavelength region. More specifically, one aspect of the present disclosure is related to the apparatus for treatment and/or prophylaxis of the retinal degeneration such as the age-related macular degeneration and the hereditary retinal degeneration by light stimulation. In some embodiments, the apparatus for treatment and/or prophylaxis of the retinal degeneration such as the age-related macular degeneration and the hereditary retinal degeneration according to the present disclosure may have at least one light source which emits light and a driving circuit which drives the light source. Here, the light emitted by the light source is light of a specific wavelength region which produces an effect of treatment and/or prophylaxis of the retinal degeneration such as the age-related macular degeneration and the hereditary retinal degeneration by being irradiated to the living body.

Moreover, one aspect of the present disclosure is related to the apparatus for treatment and/or prophylaxis by light stimulation. In some embodiments, the apparatus for treatment and/or prophylaxis of at least one symptom selected from the group consisting of low vision, metamorphopsia, central scotoma, and complete loss of vision according to the present disclosure may have at least one light source which emits light and a driving circuit which drives the light source. Here, the light emitted by the light source is light of a specific wavelength region which produces an effect of accelerating an improvement or suppression of at least one symptom selected from the group consisting of low vision, metamorphopsia, central scotoma, and complete loss of vision by being irradiated to a living body.

In the present specification, the apparatus for treatment and/or prophylaxis of the retinal degeneration such as the age-related macular degeneration and the hereditary retinal degeneration by light stimulation and the apparatus for treatment and/or prophylaxis of at least one symptom selected from the group consisting of low vision, metamorphopsia, central scotoma, and complete loss of vision by light stimulation are sometimes collectively referred to as 'apparatus for controlling biological function', or simply 'apparatus'.

These apparatuses, by controlling the irradiation state of light, are considered to be capable of applying stimulation to the macula at a central portion of the retina, and particularly to the choroidal coat under the macula. As described above, the improvement or suppression of at least one symptom selected from the group consisting of low vision, metamorphopsia, central scotoma, and complete loss of vision is accelerated in the patient having received the light stimulation, thereby leading to treatment and/or prophylaxis of the retinal degeneration such as the age-related macular degeneration and the hereditary retinal degeneration.

In some embodiments, the apparatus according to the present disclosure, as described above, is an apparatus for treating and/or preventing the retinal degeneration such as the age-related macular degeneration and the hereditary retinal degeneration by accelerating the improvement or suppression of at least one symptom selected from the group consisting of low vision, metamorphopsia, central scotoma, and complete loss of vision by irradiating the living body with violet light continuously or pulsed at a specific frequency, and is characterized by including a light source which emits the violet light and a light-emission cycle controller which makes the light be continuously irradiated or pulsed at a specific frequency, and a light-emission time controller which makes irradiate the violet light at a specific time or for a specific period of time, and by being an apparatus used for treatment or prophylaxis of the disorder.

Moreover, the apparatus according to the present disclosure, in some embodiments, is an apparatus which controls a biological function related to the retinal degeneration such as the age-related macular degeneration and the hereditary retinal degeneration by irradiating the living body with violet light continuously, and is characterized by including the light source which emits the violet light and the light-emission time controller which makes irradiate the violet light at a specific time or for a specific period of time.

Moreover, the apparatus according to the present disclosure, in some embodiments, is related to the abovementioned apparatus used for treatment and/or prophylaxis of the disorder by light stimulation which includes at least one light source which emits light, and a driving circuit which drives the light source, and the light emitted by the light source is light of a specific wavelength region which produces the effect by being irradiated to a living body. Therefore, one aspect of the present disclosure is related to the apparatus for treatment and/or prophylaxis of the retinal degeneration such as the age-related macular degeneration and the hereditary retinal degeneration by light stimulation which is configured to include at least one light source which emits light and the driving circuit which drives the light source, and the driving circuit includes at least one processor which is communicably connected to the light source and at least one memory, and at least one memory for storing commands that are executable by the processor, and the light source emits light of a specific wavelength region which produces an effect of treatment and/or prophylaxis of retinal degeneration such as age-related macular degeneration and hereditary retinal degeneration by being irradiated to a living body. Moreover, one aspect of the present disclosure is related to an apparatus for treatment and/or prophylaxis of at least one symptom selected from the group consisting of low vision, metamorphopsia, central scotoma, and complete loss of vision by light stimulation which is configured to include at least one light source which emits light and the driving circuit which drives the light source, and the driving circuit includes at least one processor which is communicably connected to the light source and at least one memory, and at least one memory which is for storing commands that are executable by the processor, and the light source emits light of a specific wavelength region which produces an effect of treatment and/or prophylaxis of at least one symptom selected from the group consisting of low vision, metamorphopsia, central scotoma, and complete loss of vision by being irradiated to a living body.

### (Light source)

The wavelength of light emitted by the light source is not restricted in particular, and in some embodiments, violet light defined by a wavelength range of 360 nm to 400 nm is used.

A light source with an oscillating frequency in a range of 0 Hz (continuously irradiated light, direct light) to 150 Hz can be used preferably. The frequency can be adjusted in units of 0.5 Hz and 1 Hz by setting the controller, and light of an arbitrary blinking frequency can be produced. As the blinking frequency is increased, there is an advantage that the blinking is not sensed any more, although this perception varies from individual to individual. The blinking frequency is not restricted to 10 Hz or 60 Hz used in experimental examples.

The irradiance of light from the light source may be variable or may be a constant value. Although irradiance with maximum output of 310 µW/cm² is used in some embodiments, it is not restricted to this value. The light source can be configured arbitrarily for irradiance within a range of 0.1 µW/cm² (0.001 W/m²) to 5000 µW/cm² (50 W/m²) for example, or irradiance within a range of 1 µW/cm² (0.01 W/m²) to 1000 µW/cm² (10 W/m²) for example, or irradiance within a range of 0.5 µW/cm² (0.005 W/m²) to 500 µW/cm² (5 W/m²) for example, or irradiance within a range of 0.5 µW/cm² to 1000 µW/cm². Furthermore, since a light source with such irradiance can be used easily for spectacles, spectacle frame, and other portable irradiating apparatuses, it can be worn even in day-to-day life. Even with weak light of extremely small amount (light with weak optical intensity) in particular, an occurrence of a characteristic phenomenon has been verified, and an effect on various parts of a living body including choroid coat under the macula at a central portion of the retina and an effect on cell activity (used with a significance including gene expression control) can be anticipated.

Light may be specified in terms of relative luminosity. Since characteristics of the present invention can be realized even with a low relative luminosity, the violet light which generates stimulation in a living body can be irradiated while blinking at a low relative luminosity, and a desired site can be stimulated without strain on a living body.

It is preferable to set the irradiation time of light arbitrarily in accordance with the purpose thereof, and it may be a short time or a long time. The light can arbitrarily be irradiated intermittently (at regular interval or irregular interval) or continuously. The time of irradiation of light can be set to be a time slot from 4 pm to 9 pm, from 5 pm to 8 pm, or from 6 pm to 7 pm, and the time period can be set to be at least 10 minutes, at least 15 minutes, at least 20 minutes, at least 30 minutes, at least one hour, at least two hours, at least three hours, at least four hours, or at least five hours. In some embodiments, irradiation for a period of five hours from 4 pm to 9 pm, irradiation for a period of three hours from 5 pm to 8 pm, or irradiation for a period of one hour from 6 pm to 7 pm is used. In a case of irradiation in which the time has been set in this manner, a timer function can be used.

The light source may be spectacles with a light source or a spectacle frame with a light source. Such spectacles or spectacle frame being easy to wear, and having a light source which emits light at a blinking frequency fitted to spectacles or spectacle frame with no uncomfortable feeling on a daily basis, they are highly practical, and can be worn constantly in various situations and varied environments. Moreover, the light source may be a desktop light source, or a light source installed nearby or in front of face such as mobile terminal mounted light source, or a non-mounting type light source such as a portable light source, or may be a mounting type light source such as an indoor lighting, a desk lamp, and a dedicated device, and can be an apparatus in various forms of light source appropriate for an environment of usage.

### (Controller)

The controller is a section which controls irradiation state (irradiate continuously or at a blinking frequency) of light from the light source. The controller may be provided with an electric power source for supplying electric power to the light source, and such electric power source may be a battery, or may have a wire drawn to a battery installed at a different location. Moreover, in a case of being immovable at one location, it may be connected to a household electric power supply.

It is preferable that the controller, by transceiving to and from an isolated controller such as a portable terminal, changes irradiation conditions of light such as blinking frequency of light, irradiance, irradiation time, irradiation-start time, and irradiation-end time. Since such controller carries out an isolated control of various irradiation conditions mentioned above, it is possible to have the desired effect by setting arbitrarily the irradiation conditions appropriate for controlling the desired biological function.

Furthermore, the controller may have controller functions and timer functions of the light source. The controller functions include functions such as making the frequency and irradiance variable, and setting irradiation time. Moreover, the timer functions include an ability to set the irradiation time. Such controller functions and timer functions may have been provided integrally with the instrument, or may be provided as separate members.

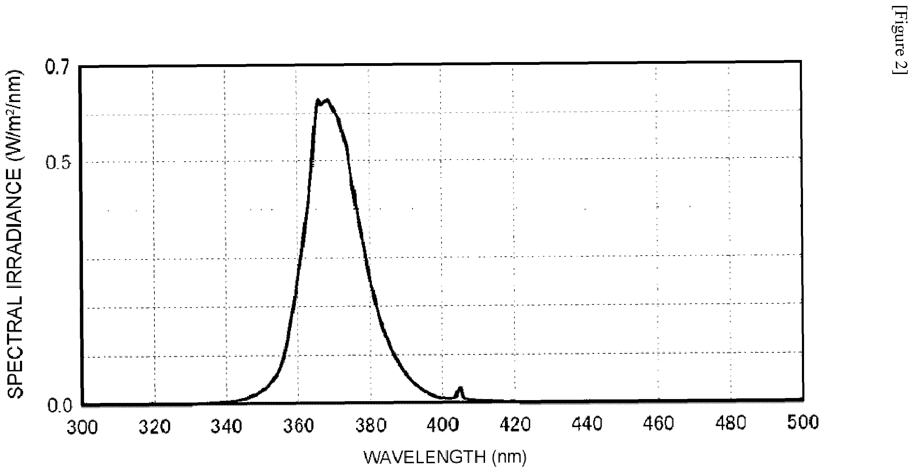

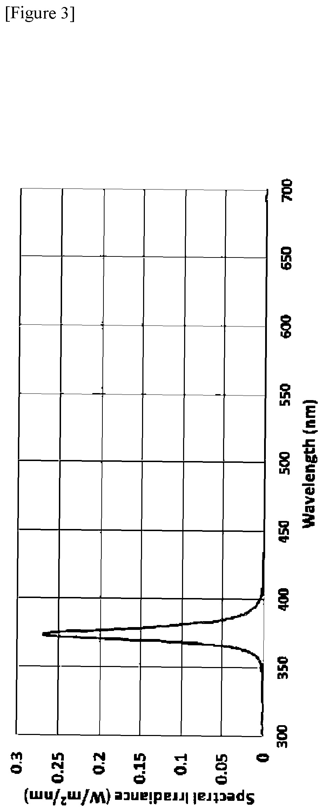

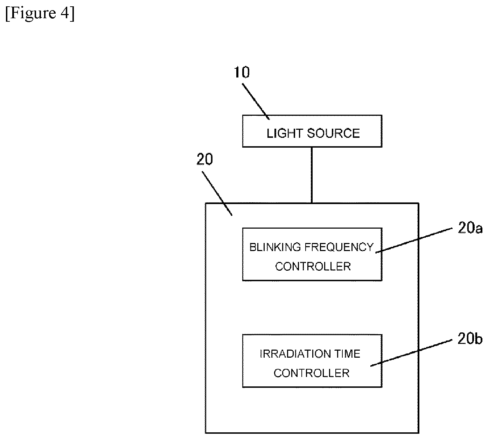

A block diagram of a simplified example of an apparatus which can be used for the abovementioned control of biological functions as an embodiment of the apparatus according to the present disclosure is shown in Fig. 4. The apparatus shown in Fig. 4 may include various functions of the apparatus for treatment and/or prophylaxis of retinal degeneration such as age-related macular degeneration and hereditary retinal degeneration by light stimulation and the apparatus for treatment and/or prophylaxis of at least one symptom selected from a group consisting of low vision, metamorphopsia, central scotoma, and complete loss of vision by light stimulation (these apparatuses can be collectively referred to as 'apparatus for controlling biological function') described in the present specification, and therefore, all the abovementioned apparatuses in the present specification can be represented by the block diagram in Fig. 4. The apparatus for controlling biological function can have a light source 10 and a controller 20. The light source 10 irradiates light of a specific wavelength region. It is preferable that a wavelength of light irradiated by the light source 10 includes VL (violet light) or light in a wavelength range of 360 nm to 400 nm described heretofore, and more preferably, includes of a wavelength of 380 nm. The light source 10 may be an arbitrary light source, and a light emitting diode (LED) can be used preferably from viewpoints such as small size, long life, and ease of blinking control (refer to Fig. 2 for an example of a spectrum of violet fluorescent light and Fig. 3 for an example of spectrum of an LED). The number of light sources 10 may be one or may be plural depending on factors such as an irradiance and an intended range of irradiation of the light source.

The controller 20 is connected to the light source 10 by a wired connection or a wireless connection, and is configured to control irradiation conditions of the light source 10. The irradiation conditions can include at least one of the blinking frequency and irradiation time of the light source 10, and therefore, the controller 20 can include at least one of a blinking frequency controller 20a and an irradiation time controller 20b. The blinking frequency may be preferably 0 Hz or a frequency in a range of 30 Hz to 75 Hz, more preferably 0 Hz or a frequency in a range of 35 Hz to 45 Hz, and particularly more preferably 0 Hz or 40 Hz. The blinking frequency of 0 Hz refers to continuously irradiated light. The irradiation time can be set arbitrarily in a range of 10 seconds to 24 hours per day for example, and a specific period of time for which the irradiation is to be continued can be set arbitrarily to a period of one day to few years, or more than that for example.

The controller 20 can include a processor such as a CPU (Central Processing Unit), and executes a processing of controlling irradiation conditions of the light source 10. The processing carried out by the controller 20 may be realized by a computer program or may be realized by hardware by a logic circuit. The computer program may be stored in a computer-readable recording medium. The recording medium having the computer program recorded may be a non-transient recording medium. The non-transient recording medium is not restricted in particular, and may be a recording medium such as a memory card and a CD-ROM (Compact Disc - Read Only Memory). The computer program stored in the recording medium can be installed in a computer unit via an appropriate reader. Examples of appropriate reader include a card reader in a case in which the recording medium is a memory card and a CD (Compact Disc) drive in a case in which the recording medium is a CD-ROM. Moreover, the computer program may be a computer program downloaded to a computer unit via a communication network from an external server.

In the apparatus according to the present disclosure, the driving circuit may include at least one processor which is communicably connected to the light source and at least one memory, and at least one memory for storing commands that are executable by the processor.

The light source 10 of the apparatus for controlling biological function shown in Fig. 4 may be spectacles with a light source or a spectacle frame with a light source, a desktop light source, a mobile terminal mounted light source, a light source installed nearby or in front of face, a portable light source, an indoor lighting, or a desk lamp. Moreover, the apparatus for controlling biological function may be provided as a product with a light source including spectacles or a spectacle frame, a desktop light, a mobile terminal, a case for mobile terminal, a head-worn article (such as cap, earphone headphone), a portable light, an indoor lighting, or a desk lamp, having at least the light source 10 out of the light source 10 and the controller 20 mounted thereon.

As described heretofore, the apparatus for controlling biological function by light stimulation according to the present disclosure, by irradiating a living body with light of a specific wavelength region such as violet light continuously or pulsed at a specific frequency, can lead to treatment and/or prophylaxis of retinal degeneration such as age-related macular degeneration and hereditary retinal degeneration and treatment and/or prophylaxis of at least one symptom selected from a group consisting of low vision, metamorphopsia, central scotoma, and complete loss of vision.

Moreover, one aspect of the present invention is related to a method of operating apparatus for controlling biological function by light stimulation. Therefore, some embodiments of the present disclosure are related to a method of operating apparatus which is used for controlling the biological function, or in other words, for treatment and/or prophylaxis of retinal degeneration such as age-related macular degeneration and hereditary retinal degeneration and for treatment and/or prophylaxis of at least one symptom selected from a group consisting of low vision, metamorphopsia, central scotoma, a complete loss of vision, and the apparatus includes a light source which irradiates a living body with light of a specific wavelength region continuously or pulsed at a specific frequency and a controller which controls the irradiation of the light source, for example, controls the blinking frequency, and the apparatus irradiates a living body with light of a specific wavelength region continuously or pulsed at a specific frequency. Here, the blinking frequency of the light source may be controlled to be 0 Hz or to a frequency in a range of 30 Hz to 75 Hz, and the light source may be a light source which irradiates light of a wavelength in a range of 360 nm to 400 nm. In addition, one aspect of the present invention is related to a computer program which causes an apparatus including a light source which irradiates a living body with light of a specific wavelength region continuously or pulsed at a specific frequency, and a controller which controls the irradiation of the light source, for example, controls the blinking frequency, to execute the method of operating.

The computer program according to the present disclosure may have a command stored in a non-transitory computer-readable medium. The computer program according to the present disclosure can execute predetermined steps when the command is executed by the processor. Therefore, one aspect of the present disclosure is related to a computer-readable medium which is a non-transitory computer-readable medium having commands stored therein, and as the command is executed by a processor, it is capable of executing for an apparatus including a light source which irradiates a living body with light of a specific wavelength region continuously or pulsed at a specific frequency, and a controller which controls the irradiation of the light source, for example, controls the blinking frequency, a step of operating the apparatus to control the blinking frequency of the light source to be 0 Hz or to be a frequency in a range of 30 Hz to 75 Hz, and a step of operating the apparatus such that the light source irradiates a living body with light of a wavelength in a range of 360 nm to 400 nm.

Furthermore, one aspect of the present invention is related to an instrument for treatment and/or prophylaxis of retinal degeneration such as age-related macular degeneration and hereditary retinal degeneration by light stimulation, and the instrument may include a glass, a spectacle lens, or a contact lens which allows violet light to pass through. By using such instrument, it is possible to have a favorable effect on a living body such as improvement and/or suppression of at least one symptom selected from a group consisting of low vision, metamorphopsia, central scotoma, and complete loss of vision. By using such instrument, treatment and/or prophylaxis of retinal degeneration such as age-related macular degeneration and hereditary retinal degeneration may be possible.

### [Component]

Furthermore, one aspect of the present invention is related to a component which is built-in or attached to the apparatus or the instrument, and is characterized by being replaceable. The examples of the component include components such as light source, controller which controls the emission of light irradiated, peripheral equipment, and battery.

### [Method or system for replacement, repair, and maintenance]

Furthermore, one aspect of the present invention is related to a method or a system for replacing, repairing, or carrying out maintenance of the apparatus, instrument, or the component.

### Examples

### Example 1. Test of measuring volume of CNV (choroidal neovascularization)

Fluorescent light of 5000 Kelvin of about 50 Lux was irradiated as background light to six-week-old mice (C57BL6/J, CLEA Japan Inc.) of a control group from 8 am up to 8 pm every day. For violet group, violet light (LED light source: manufactured by NICHIA CORPORATION, model no. NSPU510CS, peak wavelength: 375 nm) of irradiance 400 µW/cm² at a wavelength in a range of 360 nm to 400 nm was irradiated in addition to the background light from 5 pm up to 8 pm every day.

Seven days after the start of violet light irradiation, mice were anesthetized by injecting a mixture of three anesthetics (domitor + midazolam + Vetorphale (0.75 mg/kg + 4 mg/kg + 5 mg/kg)), and pupils were dilated by using 5% tropicamide and 5% phenylephrine hydrochloride. For each eye, laser-spot irradiation (170 mW, 75 µm, 100 ms) was carried out at three to five locations surrounding the optic nerve and CNV (choroidal neovascularization) model mice that are animal models of exudated age-related macular degeneration were prepared. Rupture of Bruch's membrane was verified by air bubbles generated at the time of laser irradiation. After the treatment of laser photocoagulation, the mice were awakened by administering anti-sedan and were returned to rearing cage. Even after this, the irradiation of violet light was continued under the abovementioned conditions.

Mice were euthanized 14 days after the start of violet light irradiation, and ocular globes were extracted and were fixed for one hour in 4% paraformaldehyde. After removing anterior eye part and neural retina, radial incision was performed and a compound body of retinal pigment epithelium and choroid coat was flattened to prepare choroid flat mount. After incubation for 15 minutes with PBS (phosphate buffered saline) containing 0.05% Triton (registered trademark) X-100, it was incubated with 5% BSA (bovine serum albumin). Thereafter, it was incubated overnight with fluorescently-labelled isolectin-B4 at 4°C. The choroid flat mount was observed with a confocal laser microscope (SP5, Leica), and volume of CNV (choroidal neovascular lesions) was measured by an image analysis software IMARIS (Oxford Instruments) from images captured.

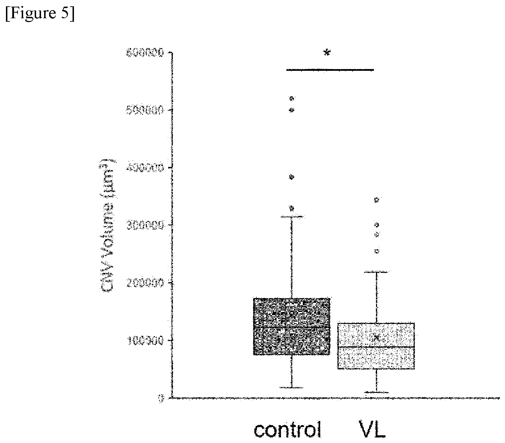

Result of measurement of the volume of CNV is shown in Fig. 5. A vertical axis indicates the volume of choroidal neovascular lesions. As shown in Fig. 5, in a violet light group (VL) subjected to violet light irradiation, the volume of CNV decreased significantly as compared to mice in a control group (control) which was not subjected to violet light irradiation. From this, it was revealed that by irradiation of violet light, it is possible to treat age-related macular degeneration, and particularly exudated age-related macular degeneration.

Moreover, from a fact that the volume of CNV decreased significantly in the violet light group to which violet light was irradiated even before preparing CNV (choroidal neovascularization) model mice, it was revealed that it is possible to prevent age-related macular degeneration, and particularly, exudated age-related macular degeneration by irradiating violet light even before contracting age-related macular degeneration.

Thus, it was revealed that it is possible to treat and/or prevent age-related macular degeneration, and particularly exudated age-related macular degeneration, by irradiation of violet light.

### Example 2, Test using mouse photodamage model

Fluorescent light of 5000 Kelvin of about 50 Lux was irradiated as background light to 10-week-old mice (BALB/c, CLEA Japan Inc.) from 8 am up to 8 pm every day, and for a violet light group, violet light (LED light source: manufactured by NICHIA CORPORATION, model no. NSPU510CS, peak wavelength: 375 nm) of irradiance 400 µW/cm² at a wavelength in a range of 360 nm to 400 nm was irradiated in addition to the background light from 5 pm up to 8 pm every day.

Mouse cage was shifted to a dark place from 8 pm on the seventh day from the start of violet light irradiation. After 14 hours of dark adaptation, the pupils were dilated by using 5% tropicamide and 5% phenylephrine hydrochloride under red lamp. The mouse cage was placed under white LED of 2500 Lux, and light was irradiated for one hour.

After the end of light irradiation, the mice were returned to a rearing cage under a normal light source of 50 lux. Even after this, the irradiation of violet light was continued under the abovementioned conditions.

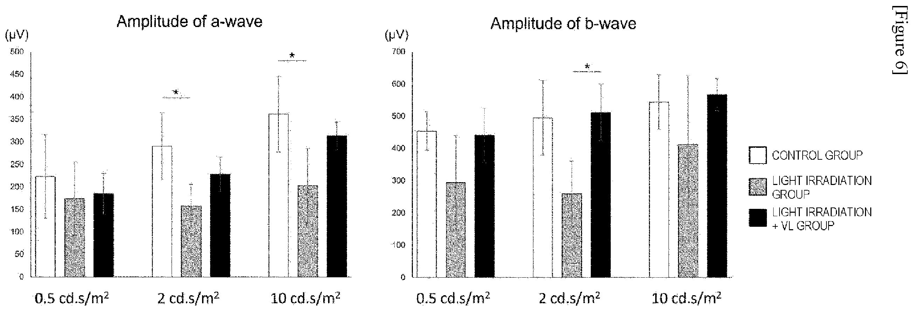

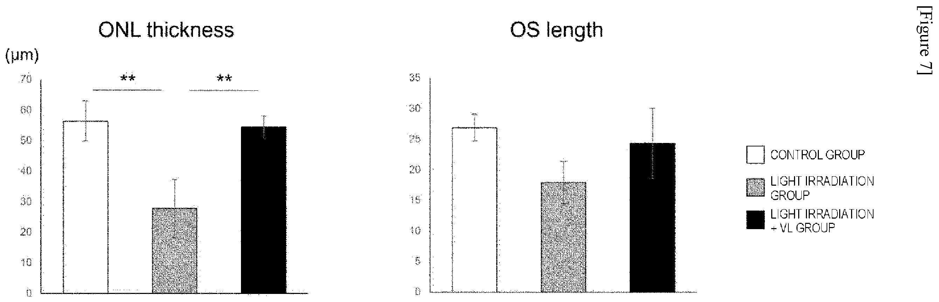

The mouse cage was shifted to a dark place from 8 pm on the tenth day from the start of violet light irradiation. After 14 hours of dark adaptation, mice were anesthetized by injecting a mixture of three anesthetics (domitor + midazolam + Vetorphale (0.75 mg/kg + 4 mg/kg + 5 mg/kg)), and the pupils were dilated by using 5% tropicamide and 5% phenylephrine hydrochloride. After pupillary dilation, an electroretinogram was measured (Fig. 6) by using a full-field light stimulation apparatus for small animals and evoked-response recording apparatus (PuRec, Mayo). The mice were euthanized after the measurement, and ocular globes were extracted and embedded in an OCT compound, thereby preparing a frozen block. A frozen section of 10 um thickness was prepared from a cross-section that included optic papilla from a block of a right eye of each mouse. Thereafter, fluorescent immunostaining using anti-rhodopsin antibody and DAPI was carried out. The section was captured by a fluorescent microscope (BZ9000, KEYENCE), and from the images captured, a thickness of an outer nuclear layer (ONL) of retina and a length of a photoreceptor cell outer segment (OS) were measured by using an image analysis software Leica Application Suite X version 3.7.4.23463.(Fig. 7).

Result of measurement of electroretinogram for each of the irradiation intensity of 0.5 cd.s/m², 2 cd.s/m², and 10 cd.s/m² is shown in Fig. 6. Left side is an amplitude of a-wave, and right side is an amplitude of b-wave. In a light irradiation group, while the amplitude decreases as compared to the amplitude in a control group, tendency of a recovery of the decrease in the amplitude was observed in a light irradiation group + VL group to which the violet light was irradiated. Moreover, the control group is a group to which neither background light nor violet light was irradiated.

Result of measurement of the thickness of the outer nuclear layer (ONL) of retina and the length of the photoreceptor cell outer segment (OS) is shown in Fig. 7. While the thickness of the ONL decreases in the irradiation group as compared to the thickness in the control group, the decrease in the thickness of the ONL in the light irradiation group + VL group to which the violet light was irradiated recovered. Similarly, while the OS length decreases in the irradiation group as compared to the OS length in the control group, tendency of a recovery of the decrease in the OS length was observed in the light irradiation group + VL group to which the violet light was irradiated. The mouse photodamage model used in this example is a model in which photoreceptor cell degeneration is induced by irradiating intense visible light to the retina of a mouse, and has been widely used as a model of retinal degeneration (photoreceptor cell degeneration) such as atrophic age-related macular degeneration and hereditary retinal degeneration. A fact that a certain curative effect by violet light was observed by using this mode indicates that application as a therapy for retinal degeneration disorder such as atrophic age-related macular degeneration and hereditary retinal degeneration is possible.

### Example 3. Clinical test of prophylaxis of age-related macular degeneration

A clinical test for the prophylaxis of age-related macular degeneration is carried out by making a patient having drusen which is considered as a precursor lesion of age-related macular degeneration wear violet light glasses, and comparing a proportion of advancement of progressive age-related macular degeneration (exudative type or atrophic type) with the proportion for the control group.

### Example 4. Clinical test for patient of exudative age-related macular degeneration

A clinical test for a patient of exudative age-related macular degeneration is carried out by making a patient diagnosed with exudative age-related macular degeneration wear violet light glasses, and comparing an amount of subretinal fluid and an amount of intraretinal edema by using an optical coherence tomography (OCT) and the like with that for the control group.

### Example 5. Clinical test for patient of atrophic age-related macular degeneration

A clinical test for a patient of atrophic age-related macular degeneration is carried out by making a patient diagnosed with atrophic age-related macular degeneration wear violet light glasses, and comparing a geographic atrophy range and a range of photoreceptor cell layer loss by using an optical coherence tomography (OCT) and the like, with that for the control group.

All publications, applications, standards, and patents mentioned in present specification are herein incorporated by reference in its entirety, and in a case of contradictory terminology, the present specification will take precedence. Scope of the present invention is not restricted by specific embodiments described in the present specification. In fact, in addition to the description in the present specification, various modifications of the present invention will be apparent to the person skilled in the art from the abovementioned description and the accompanying diagrams. Such amendments are intended to fall within the scope of the appended patent request. Some or all of the embodiments may also be described as the following supplementary notes, and the disclosure of the present application is not restricted to the following supplementary notes.

## Claims

1. A method for treating and/or preventing a retinal degeneration, the method comprising:
irradiating a subject with light of a specific wavelength region by using a light irradiating apparatus.

2. The method according to claim 1, wherein the retinal degeneration is either an age-related macular degeneration or a hereditary retinal degeneration.

3. The method according to claim 2, wherein the age-related macular degeneration is either an exudative age-related macular degeneration or an atrophic age-related macular degeneration.

4. The method according to claim 2, wherein the age-related macular degeneration is associated with at least one symptom selected from a group consisting of low vision, metamorphopsia, central scotoma, and complete loss of vision.

5. The method according to claim 4, wherein at least one symptom selected from the group consisting of low vision, metamorphopsia, central scotoma, and complete loss of vision is caused by the age-related macular degeneration.

6. The method according to claim 2, wherein an element of the age-related macular degeneration is at least one selected from a group consisting of neovascular volume, amount of subretinal fluid, amount of intraretinal edema, geographic atrophy range, and range of photoreceptor cell layer loss.

7. The method according to claim 2, wherein the age-related macular degeneration is either a precursor lesion or a progressive age-related macular degeneration.

8. The method according to claim 1, wherein the light irradiating apparatus includes a light source which is capable of irradiating a subject with light of a specific wavelength region continuously or pulsed at a specific frequency, and a controller which controls irradiation of the light source.

9. The method according to claim 1, wherein the subject is either undergoing or has undergone treatment and/or prophylaxis of the retinal degeneration.

10. The method according to claim 1, wherein the specific wavelength region includes a wavelength range of 360 nm to 400 nm.

11. The method according to claim 1, wherein the light is made to pulse at a blinking frequency in a range of 30 Hz to 70 Hz.

12. The method according to claim 1, wherein the light is irradiated during daytime.

13. The method according to claim 1, wherein the light is irradiated for one hour or more.

14. The method according to claim 1, wherein the light is irradiated such that an irradiance of light incident on eyes of the subject is in a range of 0.5 µW/cm² to 1000 µW/cm².

15. The method according to claim 1, wherein the light irradiating apparatus is spectacles with a light source or a spectacle frame with a light source, a desktop light source, a mobile terminal mounted light source, a light source installed nearby or in front of face, a portable light source, an indoor lighting, or a desk lamp.

16. An apparatus for treating and/or preventing the retinal degeneration by irradiating a subject with light of a specific wavelength region.

17. The apparatus according to claim 16, wherein the apparatus irradiates light of a specific wavelength region continuously or at a blinking frequency.

18. The apparatus according to claim 16, comprising:
a controller which controls emission of light irradiated.

19. The apparatus according to claim 18, wherein the controller, by transceiving to and from an isolated controller such as a portable terminal, carries out control by changing at least any one irradiation condition selected from a group of irradiation conditions consisting of blinking frequency of the light of a specific wavelength region, irradiance, irradiation time, irradiation-start time, and irradiation-end time.

20. The apparatus according to claim 16, comprising:
a light source; and
a driving circuit which drives the light source.

21. The apparatus according to claim 20, wherein the driving circuit includes at least one processor which is communicably connected to the light source and at least one memory, and at least one memory for storing commands executable by the processor.

22. The apparatus according to claim 16, wherein the retinal degeneration is either age-related macular degeneration or hereditary retinal degeneration.

23. The apparatus according to claim 22, wherein the apparatus treats and/or prevents the age-related macular degeneration which is either an exudative age-related macular degeneration or an atrophic age-related macular degeneration.

24. The apparatus according to claim 22, wherein the age-related macular degeneration is associated with at least one symptom selected from a group consisting of low vision, metamorphopsia, central scotoma, and complete loss of vision.

25. The apparatus according to claim 24, wherein at least one symptom selected from a group consisting of low vision, metamorphopsia, central scotoma, and complete loss of vision is caused by age-related macular degeneration.

26. The apparatus according to claim 22, wherein an element of the age-related macular degeneration is at least one selected from a group consisting of neovascular volume, amount of subretinal fluid, amount of intraretinal edema, geographic atrophy range, and range of photoreceptor cell layer loss.

27. The apparatus according to claim 22, wherein the age-related macular degeneration is either a precursor lesion or a progressive age-related macular degeneration.

28. The apparatus according to claim 16, wherein the subject is either undergoing or has undergone treatment and/or prophylaxis of the retinal degeneration.

29. The apparatus according to claim 16, wherein the specific wavelength region includes a wavelength range of 360 nm to 400 nm.

30. The apparatus according to claim 17, wherein the blinking frequency is in a range of 30 Hz to 70 Hz.

31. The apparatus according to claim 16, wherein the light is irradiated during daytime.

32. The apparatus according to claim 16, wherein the light is irradiated for one hour or more.

33. The apparatus according to claim 16, wherein the light is irradiated such that an irradiance of light incident on eyes of a subject is in a range of 0.5 µW/cm² to 1000 µW/cm².

34. The apparatus according to claim 16, wherein the apparatus is spectacles with a light source or a spectacle frame with a light source, a desktop light source, a mobile terminal mounted light source, a light source installed nearby or in front of face, a portable light source, an indoor lighting, or a desk lamp.

35. The apparatus according to claim 16, wherein light of other wavelength, sound, vibrations, magnetic field, or electric field is applied in addition, together with irradiation of light of a specific wavelength region.

36. computer-readable medium which is a non-transitory computer-readable medium having commands stored therein, and which is capable of causing an apparatus according to any one of claims 16 to 35, including a light source which is capable of irradiating a living body with light of a specific wavelength region continuously or at a specific blinking frequency and a controller which controls the blinking frequency of the light source, to perform the following steps when a command is executed by a processor:
a step of operating the apparatus such that the controller controls the blinking frequency of the light source to 0 Hz or to a frequency in a range of 30 Hz to 75 Hz; and
a step of operating the apparatus such that the light source irradiates a living body with light of a wavelength in a range of 360 nm to 400 nm.

37. An instrument for treatment and/or prophylaxis of a retinal degeneration by light stimulation, comprising:
a glass, a spectacle lens, or a contact lens which allows violet light to pass through.

38. A component which is built-in or attached to an apparatus according to claim 16, and which is replaceable.

39. component which is built-in or attached to an instrument according to claim 37, and which is replaceable.

40. A system which replaces, repairs, or carries out maintenance of an apparatus according to claim 16.

41. A system which replaces, repairs, or carries out maintenance of an instrument according to claim 37.

42. A system which replaces, repairs, or carries out maintenance of a component according to claim 38 or claim 39.

43. A method for replacing, repairing, or carrying out maintenance of an apparatus according to claim 16.

44. A method for replacing, repairing, or carrying out maintenance of an instrument according to claim 37.

45. A method for replacing, repairing, or carrying out maintenance of a component according to claim 38 or claim 39.
